Europäisches Patentamt

European Patent Office

Office européen des brevets

(11) Veröffentlichungsnummer: **0 202 399**
**B1**

(12) **EUROPÄISCHE PATENTSCHRIFT**

(45) Veröffentlichungstag der Patentschrift:
**11.10.89**

(21) Anmeldenummer: **86102420.6**

(22) Anmeldetag: **25.02.86**

(51) Int. Cl.⁴: **F 16 M 11/12, G 02 B 7/00**

(54) **Tragvorrichtung für ein optisches Beobachtungsgerät.**

(30) Priorität: **13.05.85 CH 2032/85**

(43) Veröffentlichungstag der Anmeldung:
**26.11.86 Patentblatt 86/48**

(45) Bekanntmachung des Hinweises auf die Patenterteilung: **11.10.89 Patentblatt 89/41**

(84) Benannte Vertragsstaaten:
**AT CH DE FR GB IT LI NL SE**

(56) Entgegenhaltungen:
**EP–A– 0 023 003**
**DE–A– 750 431**
**DE–B– 2 320 266**
**GB–A– 2 124 320**
**MACHINE DESIGN, Band 22, Nr. 1, Januar 1950,**
**Seiten 90-92, Cleveland; H.G. CONWAY: "Straight-**
**line linkages"**

(73) Patentinhaber: **CONTRAVES AG**
**Schaffhauserstrasse 580**
**CH-8052 Zürich (CH)**

(72) Erfinder: **Heller, Rudolf**
**Köschenrütistrasse 12**
**CH-8052 Zürich (CH)**

## Beschreibung

Die Erfindung betrifft eine Tragvorrichtung für ein verstellbares Anordnen eines Gerätes, mit einem sich von einem Fussteil aus nach oben erstreckenden Ständerteil und einem von diesem quer abstehenden Armteil, an dessen freiem Endbereich das Gerät angeordnet ist, wobei der Ständerteil für die Verstellung des Gerätes in Richtung quer zu dem Ständerteil ein schwenkbares Führungsgestänge mit einem von einem Gelenk aus aufwärts ausgerichteten Teil aufweist, an dessen oberen Ende der abstehende Armteil befestigt ist.

Als Tragvorrichtung ausgebildete Stative dieser Art sind z. B. bekannt durch die DE-B-23 20 266 und die EP-B-0 023 003 des gleichen Anmelders. Der von einer Schwenkachse aus aufwärts gerichtete Teil ist dort durch einen Arm eines zweiarmigen Hebels gebildet, so dass der am freien Ende dieses Armes befestigte Stativ-Armteil durch die Schwenkbewegung auf einer Kreisbahn bewegt wird und am Gegenarm des Hebels ein Gegengewicht erforderlich ist. Für eine Horizontalbewegung des Gerätes ist es somit bei diesen bekannten Stativen erforderlich, eine Abwärtsbewegung auf der Kreisbahn durch eine Aufwärtsschwenkbewegung des Stativ-Armteiles auszugleichen. Durch das seitliche Ausschwenken des Gegenarmes mit dem Gegengewicht entgegen der Bewegungsrichtung des Gerätes haben solche Stative einen verhältnismässig grossen Raumbedarf. Ausserdem ist der Gewichtsausgleich beim Auswechseln des Gerätes gegen ein Gerät mit anderem Gewicht aufwendig, da sowohl die Position als auch die Grösse des Gegengewichts dabei zu verändern sind.

Der Erfindung liegt die Aufgabe zugrunde, eine verbesserte Tragvorrichtung der eingangs genannten Art zu finden, die sich insbesondere durch einen verringerten Raumbedarf bei gleicher Reichweite der Gerätebewegung und damit verbunden, durch ein geringeres Gesamtgewicht und somit geringere Trägheitskräfte bei der Verstellbewegung auszeichnet. Die Lösung dieser Aufgabe erfolgt aufgrund der Merkmale des Patentanspruchs 1.

Zum längst bekannten Stand der Technik zählen schwenkbar gelagerte Gestänge, an denen sich ein ausgezeichneter Punkt auf einer nahezu exakt oder exakt geraden Bahn bewegt. So hat z. B. Peaucellier 1864 ein Gestänge beschrieben, von dem eine inverse Form bekannt ist. Letzteres besteht aus vier rhombenförmig gelenkig verbundenen Stangen. Zwei gegenüberliegende Gelenke sind über zwei unter sich gleich lange, in einem gemeinsamen Drehpunkt gelagerte Stangen gestützt. Ein drittes Gelenk wird mittels einer daran angelenkten Kurbel auf einem Kreisbogenabschnitt geführt. Die Länge der Kurbel ist gleich dem Abstand der beiden Drehlager. Das vierte, freie Gelenk des Rhombus bewegt sich dadurch entlang einer Geraden. Einem derartigen Gestänge haftet für die Verwendung als Tragvorrichtung der eingangs genannten Art noch immer der Nachteil an, ein im Vergleich zum linearen Verschiebungsweg grosses Steuergestänge aufzuweisen. Eine Übersetzung mit Hilfe üblicher Vergrösserungsparallelogramme würde einen erheblichen Mehraufwand an Material und damit Gewicht bedingen. Mit einer Ausgestaltung gemäss den kennzeichnenden Merkmalen des Anspruchs 1 hingegen lässt sich dieser Nachteil überwinden.

Da die Querverstellung des Stativ-Armteils aufgrund der Erfindung auf einer nichtkreisförmigen und vorzugsweise geraden Bahn erfolgen kann, indem die Bewegung durch ein Steuergestänge geführt ist, benötigt das Führungsgestänge für diese Querverstellung keinen nach aussen ausschwenkenden Gegenarm mit einem Gegengewicht, so dass das Gewicht und der Raumbedarf entsprechend verringert sind. Hierdurch können auch ein wesentlicher Teil des Führungsgestänges und insbesondere sein Steuergestänge in einem Gehäuse des Stativ-Ständerteils eingeschlossen sein, so dass der somit verkleidete Teil des Stativs aufgrund wegfallender Oberflächenbehandlung preiswerter herstellbar ist und der Aufwand für spätere Wartungs- und Reinigungsarbeiten verringert ist. Dies hat für Anwendungen im klinischen Bereich besondere Bedeutung.

Weitere Vorteile und Ausführungsformen der Erfindung ergeben sich aus der folgenden Beschreibung eines Ausführungsbeispieles anhand der Zeichnungen. Es zeigt:

Fig. 1 eine schematische Darstellung eines Führungsgestänges für die Verstellung des Stativ-Armteils quer zum Tragvorrichtungs-Ständerteil in einer Ausgangsposition,

Fig. 2 das Führungsgestänge nach Fig. 1 in verschiedenen Schwenkpositionen,

Fig. 3 eine schematische Darstellung eines Gestänges für den Gewichtsausgleich beim Auf- und Abwärtsschwenken des Stativ-Armteils,

Fig. 4 eine schematische Schnittansicht einer erfindungsgemässen Tragvorrichtung, und

Fig. 5 die in Draufsicht dargestellte Tragvorrichtung gemäss Fig. 4.

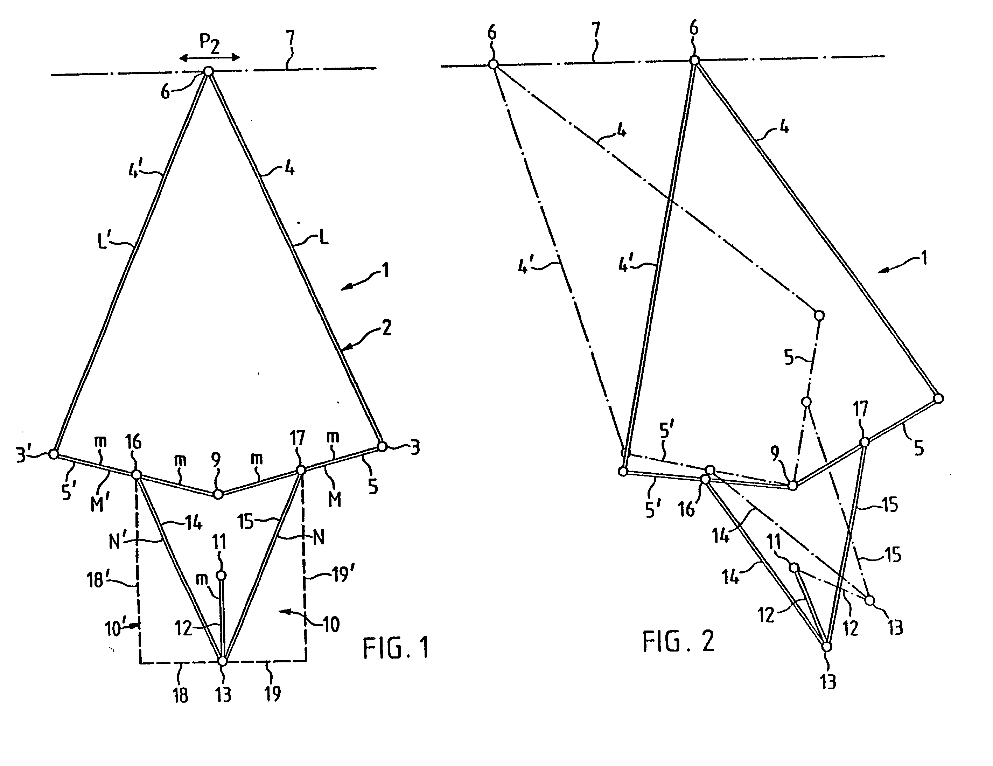

Fig. 1 zeigt ein in seiner Gesamtheit mit 1 bezeichnetes Führungsgestänge, welches ein doppelscherenartiges viergliedriges Traggestänge 2 mit jeweils zwei paarweise vorgesehenen und über ein Gelenk 3, 3' miteinander verbundenen Stangen 4, 4' und 5, 5' hat. Ein in dieser Darstellung nicht sichtbarer Stativ-Armteil ist mit geeigneten Mitteln an einem am oberen Ende der Stangen 4, 4' vorgesehenen ersten Gelenk 6 schwenkbar befestigt, und kann durch das Führungsgestänge 1 entlang einer Linie 7 in Pfeilrichtung P2 geführt werden. Die unteren, kürzeren Stangen 5, 5' des Traggestänges 2 sind an den Gelenken 3, 3' sowie an einem zweiten Gelenk 9 gelagert. Damit die Bewegung des den Stativ-Armteil tragenden ersten Gelenks 6 beim Schwenken des Führungsgestänges 1 nicht auf einer Kreisbahn verläuft, sondern geradlinig der Linie 7 folgen kann, ist das Traggestänge 2 entsprechend dem sich ändernden Abstand zwischen dem zweiten Gelenk 9 und einem Punkt auf der Linie 7, an dem sich das Gelenk 6 jeweils befindet, in seiner Länge stetig veränderbar, indem die Stangen 4, 4', 5, 5' entsprechend, durch ein angelenktes Steuergestänge 10 gesteuert und wie in Fig. 2 dargestellt, ihre Winkelposition relativ zueinander verändern.

Das Steuergestänge 10 hat eine an einem ortsfest angebrachten Gelenk 11 angelenkte Kurbel 12, deren freies Ende als Gelenk 13 ausgebildet ist. Zwei Steuerstangen 14, 15 bilden eine Verbindung zwischen dem Gelenk 13 und den zugeordneten Stangen 5, 5' des Traggestänges 2 und sind an Gelenken 16, 17 gelagert.

Die in Fig. 1 mit L,L' ; M,M' und m sowie mit N,N' bezeichneten Teile oder Teilstücke geben die Längenverhältnisse zwischen den einzelnen Stangen für die gezeichnete, bevorzugte Ausführungsform wieder. Hierbei ist der nicht näher bezeichnete Abstand zwischen den beiden Gelenken 9 und 11 gleich der Länge m der Kurbel 12 und dem Abstand m zwischen dem Gelenk 9 und den Anlenkstellen 16, 17 der Steuerstangen 14, 15 an den Stangen 5, 5' des Traggestänges 2. Die Länge M,M' der Stangen 5, 5' beträgt das Doppelte der Abstände m, während die Länge N,N' der Steuerstangen 14, 15 das 2,5-fache und die Länge L,L' der Stangen 4, 4' des Traggestänges 2 das 5-fache der Abstände m beträgt. Diese Längenverhältnisse sind Voraussetzung für die Führung des am ersten Gelenk 6 angeordneten Stativ-Armteils entlang der geraden Linie 7.

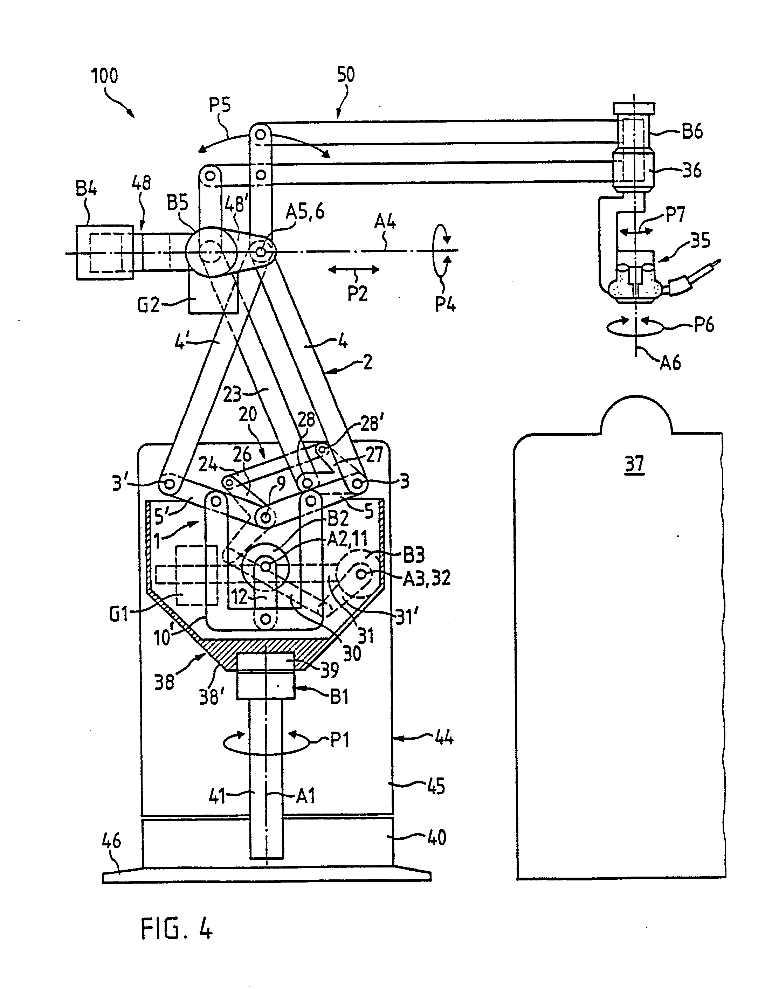

Es versteht sich, dass die einzelnen Glieder des Führungsgestänges 1 nicht aus geraden Stangen bestehen müssen, wie sie die schematischen Darstellungen der Fig. 1 und 2 zeigen, da es nur wesentlich ist, dass die Teile oder Teilstücke die Verbindung zwischen den genannten Gelenken 3, 3', 6, 9, 11, 13, 16 und 17 mit den genannten Längenverhältnissen herstellen. Beispielsweise können die Steuerstangen 14, 15 durch ein abgewinkeltes, gabelartig ausgebildetes Steuergestänge 10' ersetzt werden, wie es in Fig. 1 durch die gestrichelten Linien 18, 18', und 19, 19', sowie im bevorzugten Ausführungsbeispiel nach Fig. 4 gezeigt ist.

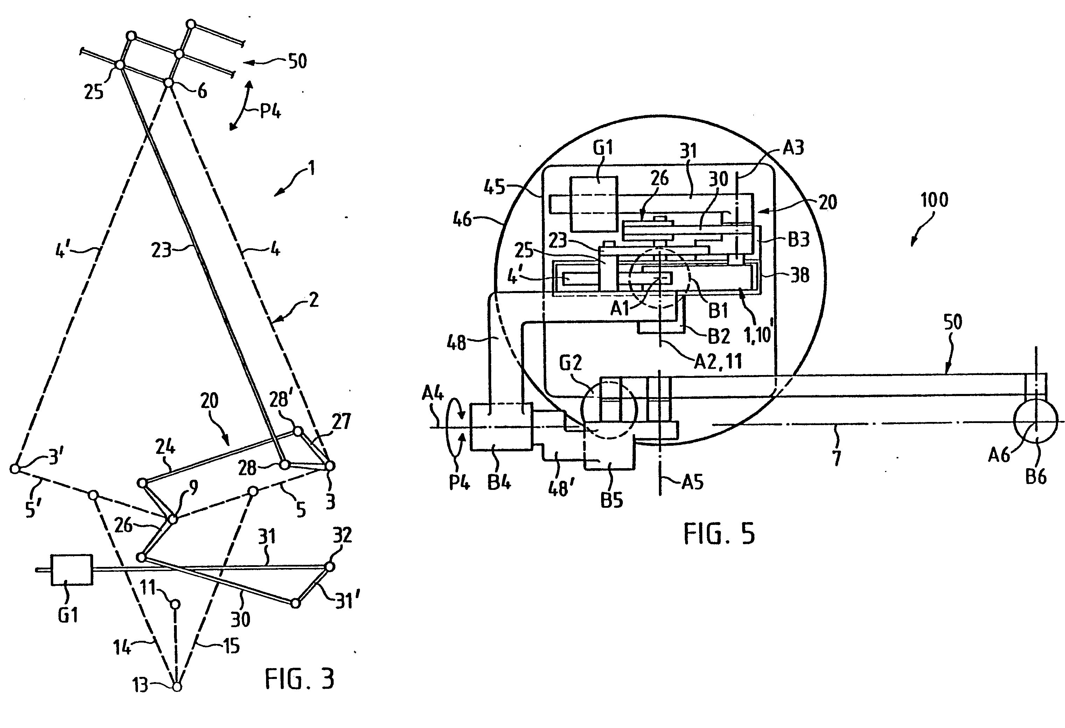

Für den Gewichtsausgleich des im wesentlichen um das erste Gelenk 6 in Pfeilrichtung P4 auf- und abwärtsschwenkbaren und mit 50 bezeichneten Stativ-Armteiles ist dem in Fig. 3 gestrichelt dargestellten Führungsgestänge 1 ein Gewichtsausgleichsgestänge 20 zugeordnet, durch das die Verbindung zwischen dem Stativ-Armteil 50 und einem ensprechend zugeordneten Ausgleichsgewicht G1 hergestellt ist. Das Gewichtsausgleichsgestänge 20 hat zwei Kopplungsstangen 23, 24, die parallel zu den gestrichelt dargestellten Stangen 4, 5 des Traggestänges 2 angeordnet sind und die Verbindung zwischen einer Anlenkstelle 25 und einem ersten Winkelhebel 26 herstellen. Der erste Winkelhebel 26 ist an der nicht näher bezeichneten Achse des zweiten Gelenks 9 gelagert. Ein zweiter Winkelhebel 27 ist an der Achse des Gelenks 3 gelagert welcher mittels Gelenke 28, 28' mit den beiden Kopplungsstangen 23, 24 wirkverbunden ist und dadurch eine gemeinsame Bewegung des Gewichtsausgleichsgestänges 20 mit dem Führungsgestänge 1 gewährleistet. Eine weitere Kopplungsstange 30 ist mit dem ersten Winkelhebel 26 und mit einem an einem ortsfesten Gelenk 32 gelagerten Winkelgestänge 31, 31' wirkverbunden. Auf der Stange 31 ist das erste Ausgleichsgewicht G1 verstellbar gelagert. Für eine ungehinderte Bewegung des durch die ausgezogenen Linien in Fig. 3 dargestellten Gewichtsausgleichsgestänges 20 mit den wirkverbundenen Teilen ist dieses beispielsweise in einer vertikalen Ebene parallel zur Ebene des durch die in Fig. 3 gestrichelten Linien dargestellten Führungsgestänges 1 angeordnet.

Die Figuren 4 und 5 zeigen in Schnittansicht und in Draufsicht ein praktisches Ausführungsbeispiel eine als Stativ ausgebildeten Tragvorrichtung 100 mit eingebautem Führungs- und Steuergestänge 1,10 sowie dem Gewichtsausgleichsgestänge 20. Die Verwendung der gleichen Bezugsziffern wie in den vorstehend beschriebenen Figuren 1 bis 3 zeigt, welche Teile der Tragvorrichtung den zuvor beschriebenen Teilen der schematischen Darstellungen entsprechen, so dass sich eine wiederholte Beschreibung erübrigt.

Der am oberen Ende des Führungsgestänges 1 angebrachte und gelagerte Stativ-Armteil 50 ist als Parallelogrammarm im wesentlichen gleich ausgebildet, wie in der genannten EP-PS-0 023 003 beschrieben ist. An seinem freien Endbereich ist ein Operationsmikroskop 35 in einem Kopfstück 36 schwenkbar befestigt. Es ist die Aufgabe der Tragvorrichtung 100 dieses Mikroskop 35 in allen Richtungen zu bewegen und in beliebigen Positionen über einen schematisch angedeuteten Operationstisch 37 zu halten, wofür sechs Freiheitsgrade der Bewegung vorgesehen sind. Jeder Freiheitsgrad wird durch eine Schwenk- oder Drehbewegung der einzelnen Elemente um eine der Achsen A1 bis A6 verwirklicht.

Um das Mikroskop 35 nach seiner Bewegung, beispielsweise von Hand, in die gewünschte Position und Ausrichtung, in dieser Position festhalten zu können, sind für die Dreh- und Schwenkbewegungen den genannten Achsen A1 bis A6, wie bei den eingangs genannten bekannten Vorrichtungen, entsprechende Magnet-Dreh- und Bremslager B1, B2, B3, B4, B5 und B6 zugeordnet. Die Freigabe dieser Magnetbremslager durch die Betätigung eines am Mikroskop vorgesehenen nichtdargestellten elektrischen Schalters ermöglicht eine Positions- und Ausrichtungsänderung des Mikroskops 35 mit geringem Kraftaufwand bei der Ueberwindung von Lagerreibungen. Der wesentlichere Kraftaufwand zur Ueberwindung von Trägheitskräften ist durch den aufgrund der Erfindung möglich gewordenen Wegfall eines Ausgleichsgewichts für die Schwenkbewegung des Führungsgestänges 1 stark verringert. Wahlweise kann jedoch ein verhältnismässig kleines zweites Ausgleichsgewicht G2 vorgesehen sein, durch das der Schwerpunkt des aus dem Mikroskop 35, dem Parallelogrammarm 50 und dem Gegengewicht G2 bestehenden Systems auf die Bewegungslinie 7 gebracht wird, so dass eine Verschiebebewegung des Mikroskops entlang der mit der Achse A4 im wesentlichen identischen Bewegungslinie 7 kein Drehmoment aufgrund von Trägheitskräften erzeugt.

Wie in Fig. 4 dargestellt, ist in einem Fussteil 40 eine die Achse A1 bildende Säule 41 in nicht näher dargestellter Weise gelagert. Am oberen Ende der Säule 41 ist das Dreh- und Bremslager B1 angeordnet, welches zur Lagerung eines mit einem Lagerteil 38' versehenen, gehäuseartig ausgebildeten Rahmens 38 ein kreiszylindrisches Kopfstück 39 aufweist. Der Rahmen 38 dient zur Lagerung des Führungs- und Steuergestänges 1, 10' sowie des Gewichtsausgleichsgestänges 20. Das Gestänge 1 und 10' ist hierbei im wesentlichen in dem Rahmen 38 angeordnet und auf der mit dem Bremslager B2 wirkverbundenen Achse A2 gelagert. Das Gewichtsausgleichsgestänge 20 ist an der mit dem Bremslager B3 wirkverbundenen Achse A3 und dem Gelenk 9 des Führungsgestänges 1 gelagert und seitlich an dem Rahmen 38 angeordnet. Das im oberen Bereich des Führungsgestänges 1 angeordnete und an Tragarmen 48, 48' gelagerte Stativ-Armteil 50 ist um die mit dem Bremslager B4 wirkverbundene Achse A4 in Pfeilrichtung P4 drehbar und um die mit dem Bremslager B5 wirkverbundene Achse A5 in Pfeilrichtung P5 schwenkbar.

Das im vorderen Bereich des Stativ-Armteils 50 angeordnete Mikroskop 35 ist um die mit dem Bremslager B6 wirkverbundene Achse A6 in Pfeilrichtung P6 drehbar und in begrenztem Bereich vorzugsweise in dem Kopfstück 36 in Pfeilrichtung P7 schwenkbar gelagert.

Ein in Fig. 4 schematisch dargestellter Ständerteil 44 weist ein der Querschnitts-Form des Fussteils 40 angepasstes Gehäuse 45 auf, das die Säule 41 sowie die Gestänge 1 und 20 bis auf eine Höhe oberhalb der Gelenke 3, 3' und 28, 28' umschliesst und in welchem der die Elemente 1, 10' und 20 tragende Rahmen 38 zusammen mit dem Stativ-Armteil 50 um die Achse A1 in Pfeilrichtung P1 drehbar ist. Der Fussteil 40 hat zur Erhöhung der Standfestigkeit des Stativs einen flach nach aussen abstehenden ringförmigen Rand 46.

In Fig. 5 ist die Tragvorrichtung 100 in schematischer Draufsicht dargestellt und man erkennt den in dem Gehäuse 45 angeordneten Rahmen 38 mit dem darin gelagerten Führungs- und Steuergestänge 1 und 10', das auf der einen Seite des Rahmens 38 angeordnete Gewichtsausgleichsgestänge 20 mit dem Gewicht G1 sowie das auf der anderen Seite des Rahmens 38 angeordnete Stativ-Armteil 50. Die Darstellung nach Fig. 5 zeigt ferner, dass der Stativ-Armteil 50 bezogen auf die vertikale Achse A1, beziehungsweise auf die Achse des Ständerteils 45 querversetzt angeordnet ist. Der Stativ-Armteil 50 wird durch die winkelförmigen Trägerarme 48, 48' gehalten, welche im wesentlichen die Verbindung mit dem Führungsgestänge 1 herstellen.

Die Verstellung der Position des Ausgleichsgewichts G1 auf der Stange 31 zur Anpassung an das Gewicht eines anderen Mikroskopes oder Gerätes kann elektromotorisch erfolgen. Auch für die Verstellung des Führungsgestänges 1, d. h., für die Ausführung von Translationsbewegungen des Mikroskopes kann ein nicht dargestellter Antrieb vorgesehen sein, dessen Drehmoment entweder an einer der Stangen 5 oder an der Kurbel 12 des Führungsgestänges 1 angreift.

## Patentansprüche

1. Tragvorrichtung (100) für ein verstellbares Anordnen eines Gerätes (35), mit einem sich von einem Fussteil (40) aus nach oben erstreckenden Ständerteil (44) und einem von diesem quer abstehenden Armteil (50), an dessen freiem Endbereich das Gerät (35) angeordnet ist, wobei der Ständerteil (44) für die Verstellung des Gerätes (35) in Richtung quer zu dem Ständerteil (44) ein schwenkbares Führungsgestänge (1) mit einem von einem Traggelenk (9) aus aufwärts ausgerichteten Traggestänge (2), an dessen oberen Ende der abstehende Armteil (50) angeordnet ist, und ein darunterliegendes, in einem Kurbelgelenk (11) gelagertes, mit dem Traggestänge (2) in zwei Stützgelenken (16, 17) verbundenes Steuergestänge (10, 10') aufweist, wobei das besagte Traggestänge (2) aus vier, paarweise gleich langen, über vier Gelenke (9, 3, 6, 3') doppelscherenartig miteinander verbundenen Stangen (5, 4, 4', 5'), und das besagte Steuergestänge (10, 10') aus einer im Kurbelgelenk (11) gelagerten Kurbel (12), deren Länge im wesentlichen gleich dem Abstand zwischen dem Kurbelgelenk (11) und dem Traggelenk (9) ist, und einem Paar, am Kurbelendgelenk (13) und je einem Stützgelenk (16, 17) angelenkten Steuerstangen (14, 15) besteht, dadurch gekennzeichnet, dass die Stützgelenke (16, 17) im unteren Stangenpaar (5, 5') des Traggestänges diese Stange (5) so in zwei Längenabschnitte unterteilen, dass das Verhältnis der Länge der Stange (5) zum Abstand des Stützgelenks (17) vom Traggelenk (9) gleich dem Verhältnis der Länge der oberen Trägerstange (4) zur Steuerstange (15) ist.

2. Tragvorrichtung nach Anspruch 1, dadurch gekennzeichnet, dass die unteren Stangen (5, 5') des Traggestänges doppelt so lang und die Steuerstangen (14, 15) 2,5 Mal so lang sind wie die Kurbel (12).

3. Tragvorrichtung nach Anspruch 1 oder 2, dadurch gekennzeichnet, dass mit dem Stativ-Armteil (50) ein Ausgleichsgewicht (G1) über ein Gewichtsausgleichsgestänge (20) verbunden ist, welches durch Winkelhebel (26, 27) in den Gelenken (9, 3) des Traggestänges (2) parallel zu diesem angekoppelt ist und aus Koppelstangen (24, 23) von jeweils gleicher Länge wie die parallel dazu liegende Stange (5, 4) des Traggestänges (2) besteht.

4. Tragvorrichtung nach Anspruch 3, dadurch gekennzeichnet, dass das Ausgleichsgewicht über einen weiteren, auf einer parallel zur Kurbelachse (11, A2) angeordneten Schwenkachse (32, A3) gelagerten Winkelhebel seitlich versetzt zum Führungsgestänge (1) angeordnet ist.

5. Tragvorrichtung nach einem der Ansprüche 1 bis 4, dadurch gekennzeichnet, dass das Traggelenk (9) und das Kurbelgelenk (11) in einem Rahmen (38) gelagert sind, der am Ende einer Säule (41) um eine vertikale Achse (A1) in dem Ständerteil (44) drehbar gelagert ist.

6. Tragvorrichtung nach einem der Ansprüche 1 bis 5, dadurch gekennzeichnet, dass das Steuergestänge (10, 10') und ein Teil des Traggestänges (2) von einem Gehäuse (45) des Ständerteils (44) umschlossen sind.

7. Tragvorrichtung nach einem der Ansprüche 1 bis 6, dadurch gekennzeichnet, dass der Fussteil (40) einen flach nach aussen abstehenden, ringförmig umlaufenden Bodenrand (46) hat.

8. Tragvorrichtung nach einem der Ansprüche 1 bis 7, gekennzeichnet durch ein im Bereich des oberen Endes des Führungsgestänges (1) angeordnetes Ausgleichsgewicht (G2), durch das der Schwerpunkt des aus diesem Gewicht, dem Stativ-Armteil (50) und dem Gerät (35) bestehenden Systems mindestens angenähert auf einer gemeinsamen Bewegungslinie (7) liegt.

## Claims

1. Supporting device (100) for an adjustable arrangement of an apparatus (35), having a stand portion (44) which extends upwards from a foot portion (40) and an arm portion (50) which stands out crosswise from the latter and at the free end region of which the apparatus (35) is arranged, in which the stand portion (44) for the adjustment of the apparatus (35) in a direction crosswise to the stand portion (44) has a pivoted guide rod system (1) with a supporting rod system (2), which is aligned upwards from a supporting link (9) and at the upper end of which the arm portion (50), which stands out, is arranged, and a subjacent control rod system (10, 10') which is mounted at a crank link (11) and is connected with the supporting rod system (2) at two support links (16, 17), said supporting rod system (2) consisting of four rods (5, 4, 4', 5') which in pairs are of the same length and are connected with each other by way of four links (9, 3, 6, 3') in the manner of double shears, and said control rod system (10, 10') consisting of a crank (12), which is mounted at the crank link (11) and the length of which is substantially equal to the distance between the crank link (11) and the supporting link (9), and of a pair of control rods (14, 15) articulated at the crank end link (13) and, respectively, a support link (16, 17), characterised in that the support links (16, 17) in the lower rod pair (5, 5') of the supporting rod system divide these rods (5) into two length sections such that the ratio of the length of the rod (5) to the distance of the support link (17) from the supporting link (9) is equal to the ratio of the length of the upper carrier rod (4) to the control rod (15).

2. Supporting device according to claim 1, characterised in that the lower rods (5, 5') of the supporting rod system are twice as long and the control rods (14, 15) are 2.5 times as long as the crank (12).

3. Supporting device according to claim 1 or 2, characterised in that a compensating weight (G1) is connected with the stand arm portion (50) by way of a weight compensating rod system (20) which by means of angle levers (26, 27) at the links (9, 3) of the supporting rod system (2) is coupled in parallel to the latter and consists of coupling rods (24, 23) of respectively the same length as the rod (5, 4) of the supporting rod system (2) lying parallel thereto.

4. Supporting device according to claim 3, characterised in that the compensating weight is arranged, by way of a further angle lever mounted on a pivot axis (32, A3) which is arranged parallel to the crank axis (11, A2), so that it is laterally offset in relation to the guide rod system (1).

5. Supporting device according to one of the claims 1 to 4, characterised in that the supporting link (9) and the crank link (11) are mounted in a frame (38) which is pivoted at the end of a column (41) about a vertical axis (A1) in the stand portion (44).

6. Supporting device according to one of the claims 1 to 5, characterised in that the control rod system (10, 10') and a portion of the supporting rod system (2) are surrounded by a housing (45) of the stand portion (44).

7. Supporting device according to one of the claims 1 to 6, characterised in that the foot portion (40) has an annularly circumferential base edge (46) which stands out outwards in a planar manner.

8. Supporting device according to one of the claims 1 to 7, characterised by a compensating weight (G2) which is arranged in the region of the upper end of the guide rod system (1) and by means of which the centre of gravity of the arrangement, consisting of this weight, the stand arm portion (50) and the apparatus (35), lies at least approximately on a common line of movement (7).

## Revendications

1. Support (100) pour recevoir de manière réglable un appareil (35) comportant une partie de bâti (44) remontant à partir d'un pied (40) et d'un élément de bras (50) en saillie transversalement par rapport à la partie de bâti et dont l'extrémité libre reçoit l'appareil (35), la partie de bâti (44) assurant le déplacement de l'appareil (35) dans une direction transversale à la partie de bâti (44) avec une tringlerie de guidage pivotante (1) et une tringlerie de support (2) alignée vers l'extérieur à partir d'une articulation de support (9), tringlerie de support dont l'extrémité supérieure porte l'élément de bras (50) en saillie ainsi qu'une tringlerie de commande (10, 10') se trouvant en-dessous, montée dans une articulation de manivelle (11) et reliée à la tringlerie de support (2) par deux articulations d'appui (16, 17), la tringlerie de support (2) se composant de quatre tiges (5, 4, 4', 5') regroupées par paires, de même longueur et reliées par quatre articulations (9, 3, 6, 3') en double ciseau, et la tringlerie de commande (10, 10') comprend une manivelle (12) reliée à l'articulation de manivelle (11), manivelle dont la longueur est pratiquement égale à la distance entre l'articulation de manivelle (11) et l'articulation de support (9), ainsi que d'une paire de tiges de commande (14, 15) articulées sur l'autre articulation (13) de la manivelle et sur une articulation d'appui (16, 17), support caractérisé en ce que l'articulation d'appui (16, 17) subdivise les tiges (5, 5') de la paire inférieure (5, 5') de la tringlerie de support en deux segments, le rapport des longueurs de la tige (5) à la distance entre l'articulation d'appui (17) et 12 de support (9) étant égal au rapport de la longueur de la tringlerie de support supérieure (4) et de la tringlerie de commande (15).

2. Support selon la revendication 1, caractérisé en ce que les tiges inférieures (5, 5') de la tringlerie de support ont une longueur double de la manivelle (12) et les tiges de commande (14, 15) ont une longueur égale à 2,5 fois celle de la manivelle.

3. Support selon la revendication 1 ou 2, caractérisé en ce qu'un contrepoids (G1) est relié par une tige d'équilibrage (20) à l'élément de bras de support (50), tige qui est associée par des leviers coudés (26, 27) aux articulations (9, 3) de la tringlerie de support (2) parallèlement à celles-ci et se composant de tiges de liaison (24, 23) ayant chaque fois la même longueur que les tiges parallèles (5, 4) de la tringlerie de support (2).

4. Support selon la revendication 3, caractérisé en ce que le contrepoids est décalé par rapport à la tringlerie de guidage (1) par un autre levier coudé monté sur un axe de pivotement (32, A3) parallèle à l'axe de manivelle (11, A2).

5. Support selon l'une des revendications 1 à 4, caractérisé en ce que l'articulation de support (9) et l'articulation de manivelle (11) sont montées sur un châssis (38) lui-même monté à rotation à l'extrémité d'une colonne (41) autour d'un axe vertical (A1) dans la partie de bâti (44).

6. Support selon l'une des revendications 1 à 5, caractérisé en ce que la tringlerie de commande (10, 10') et une partie de la tringlerie de support (2) sont entourées par un carter (45) de la partie de bâti (44).

7. Support selon l'une des revendications 1 à 6, caractérisé en ce que le pied (40) comporte un bord annulaire périphérique plat (46) en saillie vers l'extérieur.

8. Support selon l'une des revendications 1 à 7, caractérisé par un contrepoids (G2) au niveau de l'extrémité supérieure de la tige de guidage (1), pour que le centre de gravité du système formé de ce contrepoids, de l'élément de bras de support (50) et de l'appareil (35) se trouve au moins approximativement sur une ligne commune (7) de déplacement.

FIG. 1

FIG. 2

EP 0 202 399 B1

FIG. 3

FIG. 5

EP 0 202 399 B1

FIG. 4